# EUROPEAN PATENT SPECIFICATION

(11) **EP 3 060 546 B1**
(45) Date of publication and mention of the grant of the patent: **13.02.2019**
(21) Application number: 14856292.9
(22) Date of filing: 21.10.2014
(51) Int. Cl.: C07C 403/20

(54) **PROCESSES FOR THE PREPARATION OF UNSATURATED ESTERS**
VERFAHREN ZUR HERSTELLUNG VON UNGESÄTTIGTEN ESTERN
PROCÉDÉS DE PRÉPARATION D'ESTERS INSATURÉS

(30) Priority: 21.10.2013 IL 22899013
(43) Date of publication of application: 31.08.2016
(73) Proprietor: International Flavors & Fragrances Inc., New York, NY 10019 (US)
(72) Inventor: BECKER, Yigal, 6299723 Tel-Aviv (IL); CHESKIS, Michael, 3685226 Nesher (IL)
(74) Representative: Barker Brettell LLP
(86) International application number: PCT/IL2014/050911
(87) International publication number: WO 2015/059696

(56) References cited:
- WO-A1-2011/073387
- JP-A- S6 081 164
- TSUNEO KAWANOBE ET AL. NEW SYNTHESES OF (±)-AMBROX, (+)-AMBRA OXIDE AND THEIR STEREOISOMERS, AGRICULTURAL AND BIOLOGICAL CHEMISTRY 30 December 1986, pages 1475 - 1476,1478 1478, XP000561979

## Description

### TECHNOLOGICAL FIELD

The invention provides a process for the preparation of C1-4 alkyl 4-methyl-6-(2,6,6-trimethylcyclohex-1-enyl) hex-3-enoate compounds which are useful precursors for the synthesis of the amber family products such as 3a,6,6;9a-tetramethyldodecahydronaphtho[2;l-b]furan.

### PRIOR ART AND BACKGROUND OF THE INVENTION

References considered to be relevant as background to the presently disclosed subject matter are listed below:
WO2012085056 teaches that alkyl 4-methyl-6-(2,6,6-trimethylcyclohex-l-enyl)hex-3-enoate **(4)** having high E isomer content are synthesized in two steps. In the first step cited from WO2011073387, Knoevenagel condensation of 2-methyl-4-(2,6,6- trimethylcyclohex-l-en-l-yl)butanal **(1)** with dialkyl malonate **(2)** in presence of titanium tetrachloride, pyridine and a mixture of carbon tetrachloride-THF gives intermediate conjugated malonate **3.**

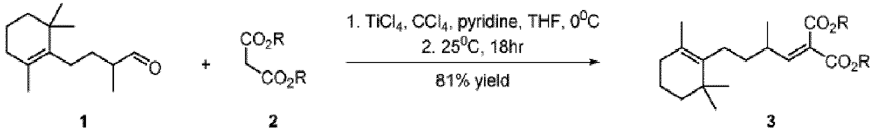

The disadvantage of this method is due to the corrosive and fuming nature of titanium tetrachloride and the use of environmentally unfriendly solvent carbon tetrachloride. In addition, pyridine and THF are water soluble and are not easily recycled.

In the following step, WO2012085056 teaches that the conjugated ester **3** is decarbalkoxylated in presence of lithium chloride and NMP (N-Methyl-2-pyrrolidone) to afford monoester **4** having high E content:

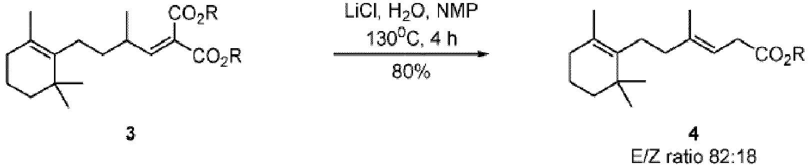

Lithium chloride can be replaced by magnesium chloride with similar results.

In a new embodiment of WO2012085056, aldehyde **1** is condensed with malonic acid (**2,** R = H) in presence of catalytically effective amount of an ammonium acetate or an amino acid such as proline, or a dipeptide such as glycyl-glycine in a mixture of tert-butanol-hexane to give intermediate conjugated malonate **3** (R = H). The solvent is then replaced by NMP, Lithium chloride or magnesium chloride is added and the mixture is heated to effect decarbalkoxylation to **4** (R = H).

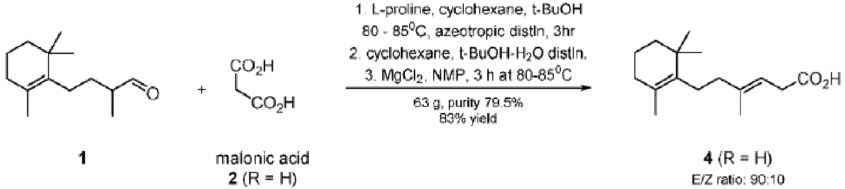

The disadvantage of this step is the need to recover three different organic solvents.

According to US7932418 conjugated ester **3** can be decarbalkoxylated to **4** (R = alkyl) in high yield, high beta-gamma selectivity and high E selectivity in presence of catalytic amount of calcium 2-ethylhexanoate. Lower E-selectivity was obtained using magnesium 2-ethylhexanoate or MgCl₂ as trans esterification catalysts.

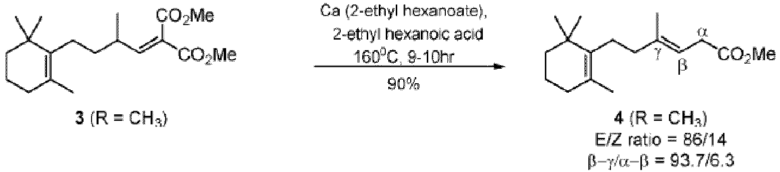

The disadvantage of this method is the use of the relatively expensive catalyst calcium 2-ethylhexanoate.

Tsuneo Kawanobe et al., New Syntheses of (±)-Ambrox, (+)-Ambra Oxide and Their Stereoisomers, Agricultural And Biological Chemistry, (1986), pages 1475 - 1480, XP000561979, discloses the synthesis of a mixture of *trans*-β-monocyclohomofarnesic acid **(7a)** and a *cis* isomer **(7b)** in an 87% yield (*ca.* 1:1). The acid mixture of **7a** and **7b** was esterified by TiCl₄/EtOH to give an ester mixture (**8a∼b**).

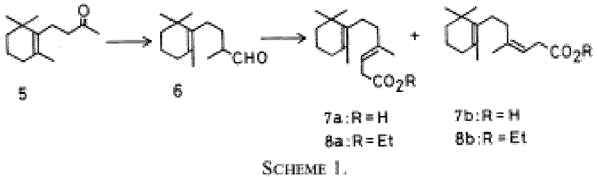

### GENERAL DESCRIPTION

The present invention provides a process for the preparation of alkyl 4-methyl-6-(2,6,6-trimethylcyclohex-l-enyl)hex-3-enoate **(4)** comprising a single step wherein 2-methyl-4-(2,6,6-trimethylcyclohex-l-en-l-yl)butanal **(1)** is reacted with monoalkyl malonate **(5);** wherein R is selected from C₁-C₄ alkyl.

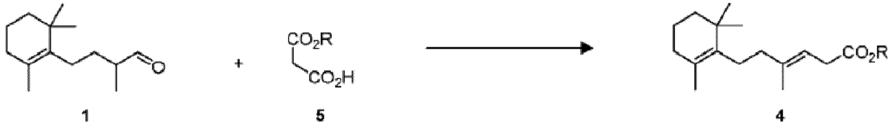

Said process of the claimed invention is catalyzed by at least one anhydrous metallic Lewis acid in at least one dipolar aprotic solvent; wherein the at least one anhydrous metallic Lewis acid is selected from MgCl₂ and LiCl.

In some embodiments said at least one dipolar aprotic solvent is selected from dimethylacetamide (DMAC), dimethylsulfoxide (DMSO), NMP (N-methyl-2-pyrrolidone) and any combination thereof.

In the claimed invention said at least one anhydrous metallic Lewis acid is selected from MgCl₂ and LiCl.

In some embodiments the process of the invention is catalyzed by MgCl₂ in the presence of dimethylacetamide as a single solvent.

In some embodiments the product of the process of the invention, alkyl 4-methyl-6-(2,6,6-trimethylcyclohex-1-enyl)hex-3-enoate **(4),** is produced in an E:Z ratio of between 82:18 to 90:10.

In some other embodiments the process of the invention is performed at a temperature of between 60°C to 120°C.

### DETAILED DESCRIPTION OF EMBODIMENTS

It has now been discovered that alkyl 4-methyl-6-(2,6,6-trimethylcyclohex-l-enyl)hex-3-enoate **(4)** having high E/Z ratio and high β-γ/α-β ratio can be synthesized in a single step by modified Knoevenagel condensation between 2-methyl-4-(2,6,6-trimethylcyclohex-l-en-l-yl)butanal **(1)** and monoalkyl malonate **(5)** in the presence of a single solvent.

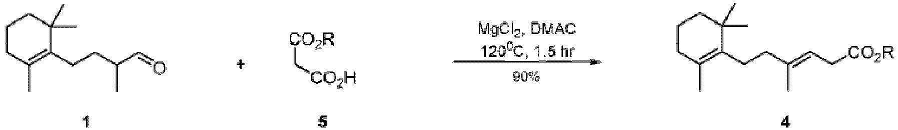

The reaction is catalyzed by anhydrous magnesium chloride (MgCl₂) in dipolar aprotic solvents such as dimethylacetamide (DMAC), dimethylsulfoxide (DMSO) or NMP (N-Methyl-2-pyrrolidone). Another Lewis acid, LiCl, was also used for the preparation of **4.**

Dimethylacetamide, being of high polarity, high stability, relatively low boiling point and low cost, is preferred.

When an amine catalyst, such as beta-alanine or L-proline, was used to catalyze the condensation the undesired conjugated ester **6** was obtained in high yield. This reaction is outside the scope of the claimed invention.

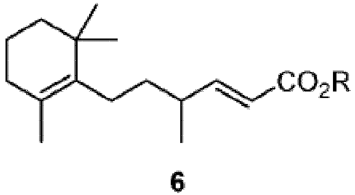

According to the reaction conditions (solvent concentration, the nature of the R group in **2** and the catalyst concentration) the E:Z ratio could vary from 82:18 to 90:10. In the claimed invention R is an alkyl group selected from C1 to C4. The preferred monoalkyl malonate esters are monomethyl malonate and monoethyl malonate.

Anhydrous magnesium chloride is readily available and is environmentally friendly. Furthermore, it was unexpectedly discovered that magnesium chloride forms a liquid complex with either NMP or DMAC which easily separates from the organic product upon cooling the reaction mixture below 60°C.

The liquid complex can be recycled, thus greatly improving the overall economics of the process. DMAC is the preferred solvent due to its relatively lower cost and lower boiling point. When DMSO is used as solvent slightly lower yields are obtained and no liquid complex is obtained.

### Example 1.

2-Methyl-4-(2,6,6-trimethylcyclohex-l-en-l-yl)butanal (1) (166g , 98% purity), dimethylacetamide (240g) and anhydrous magnesium chloride (55g) were charged to a reactor. The reactor was flushed with nitrogen and the mixture was stirred and heated to 105°C.

Then monoethyl malonate (160g, 99.5% purity) was added and the reaction mixture was stirred at 110°C for 2hr.

The reaction mixture was cooled down to 50°C, the lower dimethylacetamide-MgCl₂ liquid phase was separated, and the organic phase was washed with water (120g) to afford 227g pale yellow oil. The product consisted (GC area percentages) of 10.8% Z**-4** and 79.4% E**-4** (E: Z = 88:12). The sum of isomers yield according to internal standard was 91%.

### Example 2.

### Preparation of high-E ethyl 4-methyl-6-(2,6,6-trimethylcyclohex-l-en-l-yl) hex-3-enoate (4, R = Ethyl).

2-Methyl-4-(2,6,6-trimethylcyclohex-l-en-l-yl)butanal (1) (10 g, 79% purity), Dimethylacetamide (12 g), monoethyl malonate (8 g, Alfa Aesar 96% purity) and MgCl₂(2.75 g Sigma, anhydrous, >98%) were heated to 120°C while stirring and then stirred at this temperature for 2 hours. The yield by purity of the E-**4** isomer according to GC was 81%, and the Z-**4** isomer was 11% (the ratio E/Z = 88:12). The content of sum of isomers according to internal standard was 75%.

The reaction mixture was cooled to 60°C, the lower MgCl₂-DMAC phase was separated, the organic phase was washed with water and distilled at 120-130°C/0.1 mm.

After distillation the content of the E isomer was 71% and the Z isomer content was 9.5%.

### Example 3.

### Preparation of high-E ethyl 4-methyl-6-(2,6,6-trimethylcyclohex-l-en-l-yl)hex-3-enoate (4, R = Ethyl).

2-Methyl-4-(2,6,6-trimethylcyclohex-l-en-l-yl)butanal (1) (10 g, 79% purity), dimethylacetamide (10 g), monoethyl malonate (5.5 g) and LiCl (2 g) were heated to 130°C for 1 hour. Then monoethyl malonate (9 g) was added at 130°C for 3 hours. The reaction mixture was cooled, and stirred with water (50 g) and hexane (15 ml). The organic phase was separated and washed with 20 ml water 3 times, then the solvent was evaporated under reduced pressure to afford 12 g of the crude product containing according to GC 52% of E-**4** isomer, 9.5% of the Z-**4** isomer and 10% conjugated isomer **6.** The yield by purity of the E isomer was 59%.

### Reference Example 4.

### Preparation of high-E 4-methyl-6-(2,6,6-trimethylcyclohex-l-en-l-yl)hex-3-enoic acid (4, R = H)

2-Methyl-4-(2,6,6-trimethylcyclohex-l-en-l-yl)butanal **(1)** (10 g, 79% purity), Dimethylacetamide (12 g), malonic acid (8 g) and MgCl₂ (3 g) were heated to 130°C for 2 hours.

According to GC the reaction mixture contained 39.6% E-monocyclohomofarnesic acid (**4,** R = H) and 14.5% of the corresponding Z-**4** isomer.

### Reference Example 5.

### Preparation of Ethyl (E)-4-methyl-6-(2,6,6-trimethylcyclohex-l-en-l-yl)hex-2-enoate (6, R = Ethyl).

2-Methyl-4-(2,6,6-trimethylcyclohex-l-en-l-yl)butanal **(1)** (18 g, purity 74%), dimethylacetamide (48 g) and monoethyl malonate (12 g) were placed in the 100 ml round bottom flask and beta-alanine (0.75 g) was added. The reaction mixture was heated to 100°C and stirred at this temperature for 3 hours. Dimethylacetamide was evaporated under reduced pressure and the residue was distilled at 135-140°C/0.1 mm to give 14.7 g of a yellowish oil containing 91% of the conjugated E-**6** isomer. The yield by purity of the E-**6** isomer was 75%.

### Reference Example 6.

2-Methyl-4-(2,6,6-trimethylcyclohex-l-en-l-yl)butanal (1) (2 g, 79% purity), Dimethylacetamide (3 g), monoethyl malonate (2 g) and TiCl₄ (0.8 g) were heated to 120°C for 0.5 hour.

According to GC there was obtained 32.2% E-monocyclohomofarnesic ethyl ester (**4,** R = Ethyl), 8.3% Z-**4**-isomer and 7.5% conjugated isomer **6.**

Upon cooling of the reaction mixture no TiCl₄-DMAC complex was formed. In addition, some polymerization took place.

### Reference Example 7.

Repeating Example 5 with tin tetrachloride (SnCl₄) gave 12% E-**4** isomer and 6% Z-**4** isomer.

### Reference Example 8.

Repeating example 5 using AlCl₃ gave the conjugated isomer **6** in ∼43% yield. The yield of E-**4** isomer was only 3.7%.

## Claims

1. A process for the preparation of alkyl 4-methyl-6-(2,6,6-trimethylcyclohex-l-enyl)hex-3-enoate **(4)** comprising a single step wherein 2-methyl-4-(2,6,6-trimethylcyclohex-l-en-l-yl)butanal **(1)** is reacted with monoalkyl malonate **(5);** wherein R is selected from C₁-C₄ alkyl; 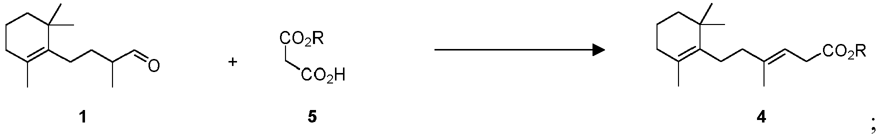
wherein the process is catalyzed by at least one anhydrous metallic Lewis acid in at least one dipolar aprotic solvent; and
wherein the at least one anhydrous metallic Lewis acid is selected from MgCl₂ and LiCl.

2. A process according to claim 1, wherein said at least one dipolar aprotic solvent is selected from dimethylacetamide (DMAC), dimethylsulfoxide (DMSO), NMP (N-methyl-2-pyrrolidone) and any combination thereof.

3. A process according to claim 1, being catalyzed by MgCl₂ in the presence of dimethylacetamide as a single solvent.

4. A process according to claim 1, wherein said product alkyl 4-methyl-6-(2,6,6-trimethylcyclohex-l-enyl)hex-3-enoate **(4)** is produced in an E:Z ratio of between 82:18 to 90:10.

5. A process according to claim 1, being performed at a temperature of between 60°C to 120°C.

## Patentansprüche

1. Verfahren zur Herstellung von Alkyl-4-methyl-6-(2,6,6-trimethylcyclohex-l-enyl)hex-3-enoat **(4)** umfassend einen einzigen Schritt, wobei 2-Methyl-4-(2,6,6-trimethylcyclohex-1-en-1-yl)butanal **(1)** mit Monoalkylmalonat **(5)** reagiert wird; wobei R aus C₁-C₄-Alkyl ausgewählt wird; 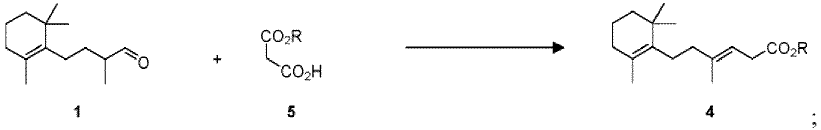
wobei das Verfahren durch mindestens eine wasserfreie metallische Lewis-Säure in mindestens einem dipolaren aprotischen Lösungsmittel katalysiert wird; und
wobei die mindestens eine wasserfreie metallische Lewis-Säure unter MgCl₂ und LiCl ausgewählt wird.

2. Verfahren nach Anspruch 1, wobei das mindestens eine dipolare aprotische Lösungsmittel unter Dimethylacetamid (DMAC), Dimethylsulfoxid (DMSO), NMP (N-Methyl-2-pyrrolidon) und irgendeiner Kombination davon ausgewählt wird.

3. Verfahren nach Anspruch 1, das durch MgCl₂ in Gegenwart von Dimethylacetamid als einzigem Lösungsmittel katalysiert wird.

4. Verfahren nach Anspruch 1, wobei das Produkt Alkyl-4-methyl-6-(2,6,6-trimethylcyclohex-l-enyl)hex-3-enoat **(4)** in einem E:Z-Verhältnis zwischen 82:18 und 90:10 hergestellt wird.

5. Verfahren nach Anspruch 1, das bei einer Temperatur zwischen 60 °C und 120 °C ausgeführt wird.

## Revendications

1. Procédé de préparation de 4-méthyl-6-(2,6,6-triméthylcyclohex-1-ényl)hex-3-énoate d'alkyle (4) comprenant une étape unique, dans lequel le 2-méthyl-4-(2,6,6-triméthylcyclohex-1-én-1-yl)butanal (1) réagit avec un malonate de monoalkyle (5) ; dans lequel R est choisi parmi alkyle en C₁-C₄ ; 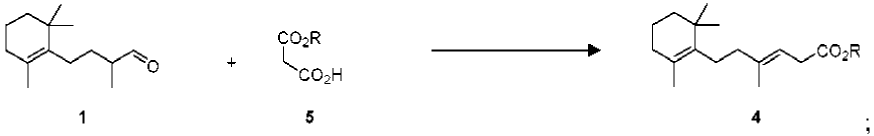
le procédé étant catalysé par au moins un acide de Lewis métallique anhydre dans au moins un solvant aprotique dipolaire ; et
dans lequel l'au moins un acide de Lewis métallique anhydre est choisi parmi MgCl₂ et LiCl.

2. Procédé selon la revendication 1, dans lequel ledit au moins un solvant aprotique dipolaire est choisi parmi le diméthylacétamide (DMAC), le diméthylsulfoxyde (DMSO), NMP (N-méthyl-2-pyrrolidone) et une combinaison quelconque de ceux-ci.

3. Procédé selon la revendication 1, étant catalysé par MgCl₂ en présence de diméthylacétamide en tant que solvant unique.

4. Procédé selon la revendication 1, dans lequel ledit produit 4-méthyl-6-(2,6,6-triméthylcyclohex-1-ényl)hex-3-énoate d'alkyle (4) est produit dans un rapport E:Z compris entre 82:18 et 90:10.

5. Procédé selon la revendication 1, étant conduit à une température comprise entre 60 °C et 120 °C.
